# EUROPEAN PATENT APPLICATION

(11) **EP 1 702 684 A2**
(43) Date of publication of application: **20.09.2006**
(21) Application number: 06250578.9
(22) Date of filing: 02.02.2006
(51) Int. Cl.: B01L 3/00, G01N 27/00, G01N 33/483

(54) **Microfluidic devices and methods of using microfluidic devices**

(30) Priority: 15.03.2005 US 80184
(71) Applicant: Agilent Technologies, Inc., Palo Alto, CA 94306 (US)
(72) Inventor: Joyce, Timothy Herbert, Loveland, CO 80537-0599 (US); Lu, Jennifer, Loveland, CO 80537-0599 (US)
(74) Representative: Ellis, Katherine Elizabeth Sleigh

(57) **Abstract**

Microfluidic devices (100), systems and methods of their use are provided. A microfluidic device (100) includes: a housing; a serpentine channel (212) disposed in the housing; and a set of resonant tunnelling electrodes disposed in the serpentine channel (212), wherein the microfluidic device (100) is configured to detect an analyte with the set of resonant tunnelling electrodes. A method for sequencing an analyte includes: aligning the analyte in a serpentine channel (212) that is disposed in a microfluidic device (100); and detecting the analyte with a set of resonant tunnelling electrodes. A system includes: a sample preparation device (120); and a detection device (140) including a serpentine channel (212) in fluid communication with the sample preparation device and a set of resonant tunnelling electrodes disposed in the serpentine channel, wherein the system is configured to detect an analyte with a set of resonant tunnelling electrodes.

## Description

This invention relates to microfluidic devices and methods of using microfluidic devices.

Microfluidic devices are becoming useful in a wide variety of applications apart from their historic uses in ink-jet printers and lab-on-a-chip assays. Potential applications include pharmaceuticals, biotechnology, the life sciences, defense, public health, and agriculture. In general, microfluidics refers to a set of technologies that control the flow of minute amounts of liquids or gases (collectively referred to as fluids) in a miniaturized system. A microfluidic device usually contains one or more channels with at least one dimension less than 1 mm. Common fluids used in microfluidic devices include whole blood samples, bacterial cell suspensions, protein or antibody solutions and various buffers. Microfluidic devices can be used to obtain a variety of measurements including molecular diffusion coefficients, fluid viscosity, pH, chemical binding coefficients, and enzyme reaction kinetics. Other applications for microfluidic devices include capillary electrophoresis, isoelectric focusing, immunoassays, flow cytometry, sample injection of proteins for analysis via mass spectrometry, PCR amplification, DNA analysis, cell manipulation, cell separation, cell patterning, and chemical gradient formation. Many of these applications are useful in clinical diagnostics. A particular application of microfluidic devices is in biopolymer analysis, for example sequencing of polynucleotides or the detection of a polynucleotide, polypeptide, or other biopolymer.

Positioning detectors in these devices has also been problematic due in part to the configuration of microfluidic channels and the size of the detectors. Moreover, aligning analytes for analysis by the detectors can also be problematic.

Alignment problems have been addressed in some applications by using conventional electrophoretic techniques to separate the analytes and prepare them for analysis. For example, gel electrophoresis can be used to stack analytes having similar charge-to-mass ratios. Although electrophoresis can effectively separate or stack analytes for analysis, electrophoresis cannot generally be employed to rapidly characterize analytes, for example in high-throughput sequence analysis.

In contrast, resonant tunnelling can be used for rapid analysis of analytes as the analytes pass near or through the electrodes of the resonant tunnelling device. Analytes must be carefully aligned for analysis by resonant tunnelling electrodes for accurate measurements because resonant tunnelling electrodes must be in relatively close proximity to one another to function effectively. Thus, the alignment of the analytes should take into consideration the distance between the resonant tunnelling electrodes. Narrowing of capillary channels to compensate for the "racetrack effect" can provide location for detection devices that must be in close proximity. Existing resonant tunnelling devices are unable to reliably and easily align analytes for analysis.

Therefore, there is a need for systems and methods for aligning analytes and characterizing them using resonant tunnelling electrodes.

According to a first aspect of the present invention, there is provided a microfluidic device as specified in claim 1.

According to a second aspect of the present invention, there is provided a method for sequencing an analyte as specified in claim 7.

According to a third aspect of the present invention, there is provided a system as specified in claim 10.

Microfluidic devices, systems, and methods of their use are provided. One exemplary device, among others, includes a housing, a serpentine channel disposed in the housing, and a set of resonant tunnelling electrodes disposed in the serpentine channel. The microfluidic device is configured to detect an analyte with the set of resonant tunnelling electrodes.

An exemplary system, among others, includes a sample preparation device, and a detection device comprising a serpentine channel in fluid communication with the sample preparation device and a set of resonant tunnelling electrodes disposed in the serpentine channel. The system is configured to detect an analyte with a set of resonant tunnelling electrodes.

An exemplary method for sequencing an analyte, among others, includes aligning the analyte in a serpentine channel that is disposed in a microfluidic device and detecting the analyte with a set of resonant tunnelling electrodes. Preferred embodiments are described below by way of example only, with reference to the accompanying drawings. Note that the components in the drawings are not necessarily to scale.
FIG. 1 is a schematic of an exemplary embodiment of a microfluidic system.
FIG. 2 is a diagram of an alternative embodiment of a microfluidic device.
FIG. 3 is a diagram of another embodiment of a microfluidic device.
FIG. 4A is a diagram of an exemplary sample preparation device.
FIG. 4B is an alternative view of a microchannel serving as a sample preparation device.
FIG. 5 is a diagram of an exemplary arcuate portion of a microchannel.
FIG. 6 is a diagram of an alternative embodiment of an arcuate portion of a microchannel.
FIG. 7 is flow diagram of an exemplary method for characterizing a biomolecule.

Prior to describing the various embodiments, the following definitions are provided where indicated.

The term "polymer" refers to a composition having two or more units or monomers attached, bonded, or physically associated to each other. The term polymer comprises biopolymers.

A "biopolymer" is a polymer of one or more types of repeating units. Biopolymers are typically found in biological systems and particularly include polysaccharides (such as carbohydrates), peptides (which term is used to include polypeptides and proteins), glycans, proteoglycans, lipids, sphingolipids, known biologicals materials such as antibodies, etc. and polynucleotides as well as their analogs such as those compounds composed of or containing amino acid analogs or non-amino acid groups, or nucleotide analogs or non-nucleotide groups. This comprises polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in hydrogen bonding interactions, such as Watson-Crick type, Wobble type and the like. Polynucleotides include single or multiple stranded configurations, where one or more of the strands may or may not be completely aligned with another. A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides. Biopolymers include DNA (including cDNA), RNA, oligonucleotides, and PNA and other polynucleotides as described in U.S. Pat. No. 5,948,902 and references cited therein (all of which are also incorporated herein by reference), regardless of the source. An "oligonucleotide" generally refers to a nucleotide multimer of about 10 to 100 nucleotides in length, while a "polynucleotide" comprises a nucleotide multimer having any number of nucleotides. A "biomonomer" references a single unit, which can be linked with the same or other biomonomers to form a biopolymer (*e*.*g*., a single amino acid or nucleotide with two linking groups one or both of which may have removable protecting groups).

"Electrophoresis" refers to the motion of a charged particle or polymer, for example colloidal particle, under the influence of an electric field. The particle will move at a velocity such that the electric force balances the viscous drag on the particle. The drag force will be determined by the hydrodynamic radius and the viscosity of the medium. The electric force is given by a hydrodynamic potential such as the zeta potential. Electrophoresis is used to separate different colloids and polyelectrolytes, as a means of analysis and to determine potentials. It can also be used to separate species with different charges (such as different proteins). The medium in which the polymers or particles move can be either a liquid, a gel, or polymer network.

"Entangled polymer solutions" refers to solutions in which polymers will interpenetrate each other. This causes entanglements and restricts the motion (reptation) of the molecules to movement along a 'virtual tube' that surrounds each molecule and is defined by the entanglements with its neighbors.

The term "gel" refers to a network of either entangled or cross-linked polymers swollen by solvent. The term is also used to describe an aggregated system of colloidal particles that forms a continuous network.

"Statistically significant" refers to a result is significant if it is unlikely to have occurred randomly. "Significant" means probably true (not due to chance). Generally, a statistically significant sequence refers to a sequence that has about a 5% or less probability of including a random sequence error.

"Serpentine channel" refers to a conduit having a curved or arcuate portion, for example a bend. Serpentine channels can include a linear and/or a non-linear portion of a conduit. An exemplary serpentine channel has a portion in the shape of an "S", "U", or "L".

Having defined some of the terms used herein, the various embodiments of the disclosure will be described.

### Exemplary Microfluidic Devices

As will be described in greater detail here, embodiments of microfluidic devices and methods of use thereof are provided. By way of example, some embodiments provide for a microfluidic device having at least one serpentine channel. As noted above, a serpentine channel is a channel having a non-linear portion or segment. The non-linear portion is typically arcuate. Channels in the disclosed microfluidic devices generally are less than about 1 mm in a given dimension, typically from about 0.1 µm to about 750 µm, more typically about 1.0 µm to about 500 µm, even more typically about 50 µm to about 250 µm in depth, width, or a combination thereof. It will be appreciated the width, depth, or combination thereof may not be uniform throughout the length of the channel. The channels can of any geometric configuration, including, but not limited to, rectangular in cross-section, tubular, or hemispheric.

One exemplary microfluidic device comprises a housing having at least one channel disposed on or in the housing. A device for detecting, monitoring, or analyzing analytes can be positioned within or tangent to the channel. In one embodiment, the housing of the disclosed microfluidic devices can be fabricated from a variety of materials, including but not limited to silicon, polydimethylsiloxane (PDMS), thermoplastic, glass, polymeric films, mylar, metal, metal alloys, or combinations thereof. The channels can be etched or bored into a surface of the device or can be formed in the interior of the device.

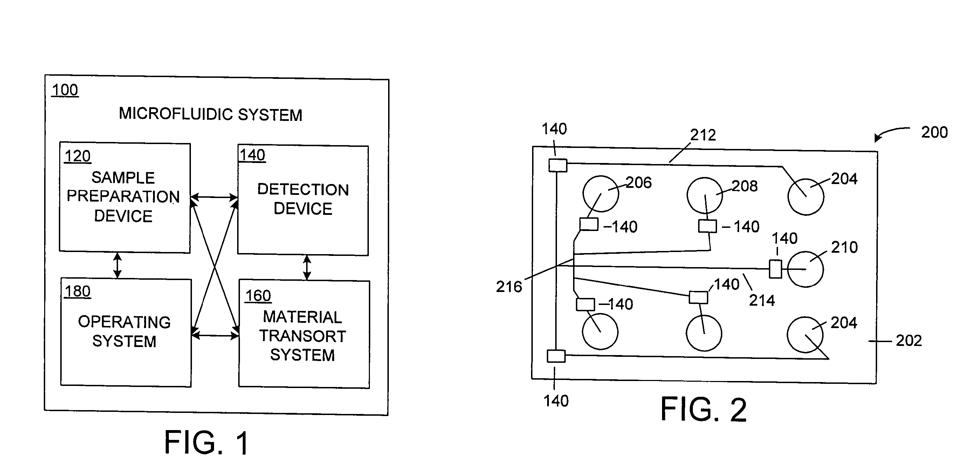

FIG. 1 shows a graphical representation of an exemplary microfluidic system 100. The microfluidic system 100 comprises a sample preparation device 120 in fluid, and optionally electrical, communication with a detection device 140. The detection device 140 comprises, but is not limited to, a set of resonant tunnelling electrodes including the electrodes 220, 222, which are in turn communicatively coupled so that data regarding an analyte such as a polymer (e.g., a target polynucleotide) can be measured, and optionally collected.

A sample is transported through microfluidic device 100 using a material transport system 160. The material transport system 160 comprises, but is not limited to, any one of electrokinetic components, electroosmotic components, electrophoretic, or other fluid manipulation components (e.g., micro-pumps and microvalves, fluid switches, fluid gates, etc.) sufficient for the movement of material within microfluidic device 100. Characteristics of the sample can be detected, monitored, and collected using the detection device 140.

The microfluidic system 100 comprises, but is not limited to, an operating system 180. The operating system 180 comprises, but is not limited to, electronic equipment capable of measuring characteristics of an analyte such as a polymer (e.g., a polynucleotide) as the polymer travels along a channel, a computer system capable of controlling the measurement of the characteristics and storing the corresponding data, control equipment capable of controlling the conditions of the detection device, and/or components that are included in the detection device 140 that are used to perform the measurements as described below. The microfluidic system 100 can also be in communication with a distributed computing network such as a LAN, WAN, the World Wide Web, Internet, and/or intranet.

The detection device 140 can measure characteristics such as, but not limited to, the amplitude or duration of individual conductance or electron tunnelling current changes as an analyte, such as a polymer, passes near or through the detection device 140. Typically, conductance occurring through an analyte or polymer as it traverses the detection device 140 is detected or quantified. More specifically, electron tunnelling conductance measurements are detected for each monomer of an analyte or polymer as each monomer traverses the detection device 140. Such measurements include, but are not limited to, changes in data which can identify the monomers in sequence, as each monomer can have a characteristic conductance change signature. For instance, the volume, shape, purine or pyrimidine base, or charges on each monomer can affect conductance in a characteristic way. Likewise, the size of the entire polynucleotide can be determined by observing the length of time (e.g., duration) that monomer-dependent conductance changes occur. Alternatively, the number of nucleotides in a polynucleotide (e.g., a measure of size) can be determined as a function of the number of nucleotide-dependent conductance changes for a given nucleic acid traversing the nanopore aperture. The number of nucleotides may not correspond exactly to the number of conductance changes, because there may be more than one conductance level change as each nucleotide of the nucleic acid passes sequentially through the detection device. However, there can be proportional relationship between the two values that can be determined by preparing a standard with a polynucleotide having a known sequence.

Components of the disclosed system include microfluidic devices. FIG. 2 shows a diagram of a representative microfluidic device 200. In this embodiment, a housing 202 comprises a plurality of wells or sample inlets 204. A sample inlet 204 is connected to a well 210 by channels 212 and 214. The well 210 can serve as a receptacle for receiving analyzed or processed samples. In operation, a sample is received into the sample inlet 204. The material transport system 160 (not shown) moves the sample through the channel 212 and throughout the microfluidic device. The channel 212 can also serve as a sample preparation device. For example, the channel 212 can comprise a separation matrix for electrophoretically sorting the sample.

The channel 212 typically is a serpentine channel having at least one linear portion and at least one non-linear portion. The non-linear portion can be a curved or arcuate portion. The curve, turn, or bend can be about 90°, less than about 90°, more than 90°, more than 180°, between 90° and 180°, or between from about 1.0° to about 90°. It will be appreciated that the channel 212 can have a plurality of bends, turns or curves. In one embodiment, a detection device (e.g., a resonant tunnelling electrode) is placed in or tangent to the arcuate portion of the channel 212.

The resonant tunnelling electrode 140 can be configured to monitor conductance, for example tunnelling current, of analytes as the analytes pass though the space separating the electrodes. The width or depth of the channel 212 is represented as W. As the channel bends or turns, the width is tapered to a constriction width CW, wherein CW < W. Generally, the width of the arcuate portion of the channel 212 will be constricted enough to reduce or eliminate analyte dispersion as a sample travels through the bend or turn. In one embodiment the width of the arcuate portion is constricted so that CW:W is about 0.75 to about 0.25, typically about 0.5. The length of the constricted portion of the channel 212 is represented as RL. In another embodiment, the arcuate portion is configured so that RL:W is greater than 1.0, and optionally CW:W is from about 0.5 to about 1.5. In still another embodiment, the channel 212 is configured so that RL:W is from about 1.0 to about 1.5. In one embodiment, the resonant tunnelling electrode can be positioned inside an arcuate portion of a channel to narrow the width of the arcuate portion to a desired amount. It will be appreciated that the turn, bend, or curve of the arcuate portion can taper to width CW sufficient for electrodes 220 and 222 of a resonant tunnelling electrode to detect changes in conductance or tunnelling current as analytes pass through the space separating electrodes 220 and 222.

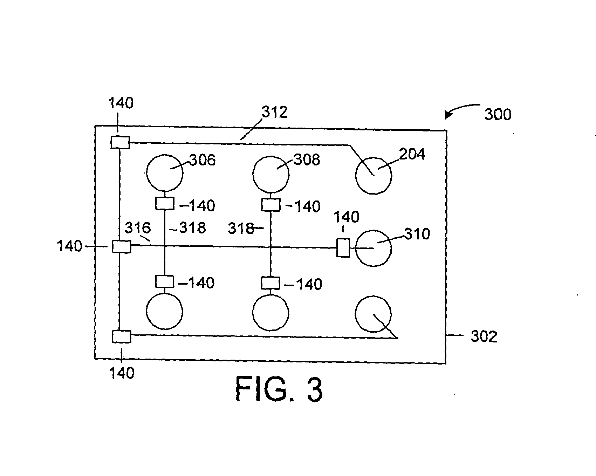

FIG. 3 shows a diagram of an alternative embodiment of the disclosed microfluidic device with alternative channel configurations. In this embodiment, a plurality of channels intersect at "T" junctions as well as optionally containing at least one turn or bend. Segments of a sample can be drawn into different channels for different analysis as the sample passes each T junction. Alternatively, different reagents can be delivered to the microfluidic device through inlets 306 or 308.

FIG. 3 shows a diagram of another representative microfluidic device 300. In this embodiment, a housing 302 includes a plurality of wells or sample inlets 204. A sample inlet 204 is connected to a well 310 by a channel 316. The well 310 can serve as a receptacle for receiving analyzed or processed samples. In operation, a sample is received into the sample inlet 204. The material transport system 160 (not shown) moves the sample through a channel 312 and throughout the microfluidic device. The channel 312 can also serve as a sample preparation device. For example, the channel 312 can include a separation matrix for electrophoretically sorting the sample.

The channel 312 typically is a serpentine channel having at least one linear portion and at least one non-linear portion. The non-linear portion can be a curved or arcuate portion. The curve, turn, or bend can be about 90°, less than about 90°, more than 90°, more than 180°, between 90° and 180°, or between from about 1.0° to about 90°. It will be appreciated that the channel 312 can have a plurality of bends, turns or curves. In one embodiment, a detection device (e.g., for example a resonant tunnelling electrode) is placed in or tangent to the arcuate portion of the channel 312.

The resonant tunnelling electrode 140 can be configured to monitor conductance, for example tunnelling current, of analytes as the analytes pass through the space separating the electrodes. The width or depth of the channel 312 is represented as W. As the channel bends or turns, the width is tapered to a constriction width CW, wherein CW < W. Generally, the width of the arcuate portion of the channel 312 will be constricted enough to reduce or eliminate analyte dispersion as a sample travels through the bend or turn. In one embodiment the width of the arcuate portion is constricted so that CW:W is about 0.75 to about 0.25, typically about 0.5. The length of the constricted portion of the channel 312 is represented as RL. In another embodiment, the arcuate portion is configured so that RL:W is greater than 1.0, and optionally CW:W is from about 0.5 to about 1.5. In still another embodiment, the channel 312 is configured so that RL: W is from about 1.0 to about 1.5. In one embodiment, the resonant tunnelling electrode can be positioned inside an arcuate portion of a channel to narrow the width of the arcuate portion to a desired amount. It will be appreciated that the turn, bend, or curve of the arcuate portion can taper to width CW sufficient for electrodes 220 and 222 of a resonant tunnelling electrode to detect changes in conductance or tunnelling current as analytes pass through the space separating electrodes 220 and 222.

### Sample Preparation Device

Prior to being analyzed by a detector, analytes, for example biomolecules, must be sorted or aligned so that data can be collected from each analyte or biopolymer. The alignment of the analytes for analysis can be achieved using the sample preparation device 120. In one embodiment, the sample preparation device 120 advantageously sorts and optionally groups or stacks similar analytes, for example polymers of a specific mass or range of masses, molecular weight, size, charge, conformation including single or double stranded conformations, or charge-to-mass ratio to be detected, for example by the resonant tunnelling electrode 140. Providing multiple polymers of similar or identical characteristics allows for collection of multiple data points for the same polymer or analyte. The multiple data points can be analyzed, for example a statistical analysis can be performed, to increase the fidelity of the result, for example determining the sequence of monomers in the polymer. Some data points may incorrectly represent a characteristic of the polymer being analyzed, for example, an incorrect sequence of monomers. Incorrect, or outlying data points can be ignored or deleted from the data set to produce a more reliable and statistically significant result.

### Sample Sorting and Stacking

In some embodiments, a plurality of analytes, for example polymers, may be sorted, stacked, or separated with the sample preparation device 120 using conventional techniques including, but not limited to, electrophoresis, capillary electrophoresis, molecular sieves, antibody capture, chromatography, affinity, polynucleotide capture, chromatography, reverse phase chromatography, and ion exchange chromatography. It will be appreciated that the sample preparation device 120 can be a channel of the microfluidic device.

In one embodiment, capillary electrophoresis can also be performed in one more microfluidic channels. A microfluidic channel comprises, but is not limited to, a surface micro-machined labyrinth, having one central inlet and at least one outlet. These separations are facilitated by the use of high voltages, which may generate electroosmotic flow, electrophoretic flow, or a combination thereof, of buffer solutions and ionic species, respectively, within the channel. The properties of the separation and the ensuing electropherogram have characteristics resembling a cross between traditional polyacrylamide gel electrophoresis (PAGE) and modern high performance liquid chromatography (HPLC). In one embodiment, electrophoretic device 120 utilizes a high electric field strength, for example, about 500 V/cm or more. One process that drives CE is electroosmosis. Electroosmosis is a consequence of the surface charge on the wall of the channel. Some channels that are typically used for separations have ionizable silanol groups in contact with the buffer contained within the channel. The degree of ionization can be controlled mainly by the pH of the buffer.

A negatively-charged channel wall attracts positively-charged ions from the buffer, creating an electrical double layer. When a voltage is applied across the channel, cations in the diffuse portion of the double layer migrate in the direction of the cathode, carrying water with them. The result is a net flow of buffer solution in the direction of the negative electrode. In untreated microchannels most solutes migrate towards the negative electrode regardless of charge when the buffer pH is above 7.0.

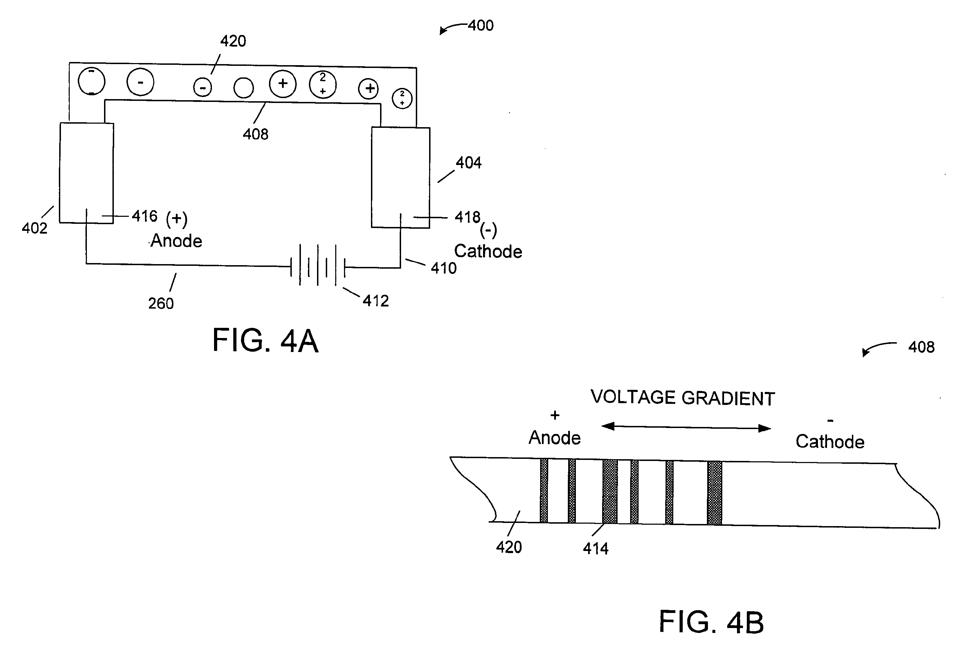

Capillary electrophoresis comprises, but is not limited to, capillary zone electrophoresis, isoelectric focusing, capillary gel electrophoresis, isotachophoresis, and micellar electrokinetic capillary chromatography. Capillary zone electrophoresis (CZE), also known as free solution capillary electrophoresis, is the simplest form of CE. The separation mechanism is based on differences in the charge-to-mass ratio. Fundamental to CZE are homogeneity of the buffer solution and constant field strength throughout the length of the capillary. Following injection and application of voltage, the components of a sample mixture separate into discrete zones 414 as shown in the microchannel 408 and sample separation matrix 420 of FIG. 4B.

With isoelectric focusing (IEF), a molecule will migrate so long as it is charged, and will stop when it becomes neutral. IEF is run in a pH gradient where the pH is low at the anode and high at the cathode. The pH gradient is generated with a series of zwitterionic chemicals known as carrier ampholytes. When a voltage is applied, the ampholyte mixture separates in the capillary. Ampholytes that are positively charged will migrate towards the cathode while those negatively charged migrate towards the anode. It will be appreciated that the pH of the anodic buffer must be lower than the isoelectric point of the most acidic ampholyte to prevent migration into the analyte. Likewise, the catholyte must have a higher pH than the most basic ampholyte.

Nucleic acids are generally electrophoresed in neutral or basic buffers as anions with their negatively charged phosphate groups. For small DNA fragments, e.g., nucleosides, nucleotides, and small oligonucleotides, free-solution techniques (CZE, MECC) can be applied―generally in conjunction with uncoated capillaries or microfluidic channels. Alternatively, separation of larger deoxyoligonucleotides can be accomplished using capillary gel electrophoresis, generally with coated capillaries or microfluidic channels, in which, as the name implies, the capillary or channel is filled with an anticonvective medium such as polyacrylamide or agarose. The gel suppresses electroosmotic flow and acts a sieve to sort analytes by size. Oligonucleotides, for example poly(dA)40-60 can be separated using this method with a gel of 8% monomer and a buffer comprising of 100 mM Tris-borate, pH 8.3 with 2 mM EDTA and 7 M urea, in under 35 min with unit base resolution.

Isotachophoresis relies on zero electroosmotic flow, and the buffer system is heterogeneous. This is a free solution technique, and the capillary or channel is filled with a leading electrolyte that has a higher mobility than any of the sample components to be determined. Then the sample is injected. A terminating electrolyte occupies the opposite reservoir, and the ionic mobility of that electrolyte is lower than any of the sample components. Separation will occur in the gap between the leading and terminating electrolytes based on the individual mobilities of the analytes.

Micellar electrokinetic capillary chromatography (MECC) is a free solution technique that uses micelle-forming surfactant solutions and can give rise to separations that resemble reverse-phase liquid chromatography with the benefits of capillary electrophoresis. Unlike isoelectric focusing, or isotachophoresis, MECC relies on a robust and controllable electroosmotic flow. MECC takes advantage of the differential partitioning of analytes into a pseudo-stationary phase including micelles. Ionic, nonionic, and zwitterionic surfactants can be used to generate micelles. Representative surfactants include, but are not limited to, SDS, CTAB, Brij, and sulfobetaine. Micelles have the ability to organize analytes at the molecular level based on hydrophobic and electrostatic interactions. Even neutral molecules can bind to micelles since the hydrophobic core has very strong solublizing power.

FIG. 4A shows a diagram of an exemplary electrophoretic device 400 using a voltage gradient or an electric field to separate various polymers or analytes. A sample inlet 402 receives a sample, for example a sample containing a plurality of polynucleotides. The sample is preferably a fluid sample in a solution buffered to a desired pH and ionic strength. Generally, the electrophoretic device has an anode buffer in an inlet 402 and a cathode buffer in an outlet 404. It will be appreciated that the pH of the buffer and ionic strength can each be modulated, which in turn can modulate the electrophoretic separation of the polymers or analytes. Additionally, viscosity builders, surfactants, denaturing agents, or other additives can be added to the sample or buffer to vary the separation resolution of the polymers. In some embodiments, capillary electrophoresis require modifications to the walls of the capillaries or channels, for example channels of fused silica. The wall can be modified in a manner to modify or suppress electroosmotic flow, and/or to reduce unfavorable wall-analyte interactions. In one embodiment, the electrophoretic device 400 does not exert electroosmotic flow on the polymers to be separated. For example, a tube or microchannel 408 can have surfaces that are neutral or uncharged during electrophoresis. Charged surfaces of the tube or microchannel 408 can optionally be coated, for example with an ionic surfactant such as a cationic or anionic surfactant. Exemplary coating substances or buffer additives include, but are not limited to, SDS, cetyltrimethylammonium bromide (CTAB), polyoxyethylene-23-lauryl ether; sulfobetaine (BRIJ), TWEEN, MES, Tris, CHAPS, CHAPSO, methyl cellulose, polyacrylamide, PEG, PVA, methanol, acetonitrile, cyclodextrins, crown ethers, bile salts, urea, borate, diaminopropane, and combinations thereof. Neutralizing charged surfaces of the channel 408 can eliminate or reduce electroosmotic flow. Alternatively, modifications to the surface of the channel 408 or to the buffers can eliminate or reverse the electroosomotic force. For example, neutral deactivation with polyacrylamide eliminates the electroosmotic flow. This results from a decreased effective wall charge and increased viscosity at the wall. Deactivation with cationic groups can reverse the electroosomotic flow, and deactivation with amphoteric molecules allows one to control the direction of the electroosmotic force by altering the pH.

A wide variety of covalent and adsorbed capillary or microfluidic channel coatings that completely suppress electroosmotic flow are known in the art and are commercially available. Coatings of covalently bound or adsorbed neutral polymers such as linear polyacrylamide are highly stable, resist a variety of analytes, and reduce electroosmotic flow to almost undetectable levels. Such coatings are useful for a wide range of applications including DNA and protein separations, with the requirement that all analytes of interest migrate in the same direction (e.g., have charge of the same sign). For many other applications, however, electroosmotic flow can be used as a pump to mobilize analytes of both positive and negative charge (e.g., a mixture of proteins with a wide range of isoelectric points), or to mobilize species with very low electrophoretic mobility. Bare fused silica capillaries exhibit strong cathodal electroosmotic flow, but are prone to adsorption of analytes, leading to irreproducible migration times and poor peak shapes.

In one embodiment, electrophoretic separation of polymers occurs in the tube, capillary, or microfluidic channel 408. The tube or microfluidic channel 408 can be coated or uncoated. In another embodiment, exemplary microfluidic channels have a diameter of less than 1 mm, typically less than about 500 µm, more typically less than about 200 µm or from about 1 µm to about 100 µm.

FIG. 4B is sectional view of the tube 408 showing bands or zones 414 of analytes as they are separated along a voltage gradient. Generally, the samples move from anode to cathode, however, it will be appreciated that the polarity can be reversed by changing the buffer system, adding ionic surfactants to the sample or buffer or coating the interior surfaces of the capillary to reduce or eliminate electroosmotic flow. Typically, the analytes in one band 414 are of uniform size, uniform number of monomers, and optionally uniform sequence.

In FIG. 4A, the power source 412 provides a voltage gradient between the anode 416 and the cathode 418. The power source 412 is in electrical communication with wells 402, 404 using conventional electrical conductors 410. Generally, the power source 412 can supply about 10 to about 60 kV, typically about 30 kV. It will be appreciated that the voltage can be adjusted to modify polymer separation. When a voltage gradient is established, individual analytes will move along the voltage gradient, for example according to their charge, mass, or charge-to-mass ratio. In conventional capillary electrophoresis, small positively charged analytes will move quickly from the anode towards the cathode. Larger positively charged polymers will follow with large negatively charged polymers traveling at the end of the sample.

During separation, the analytes travel through the channel 408. The channel 408 can be filled with a separation matrix 420 and/or a buffer to maintain ionic and pH conditions. The ionic and pH conditions can be optimized to increase the separation resolution of specific analytes. It will be appreciated that the different analytes may use different buffers, ionic concentrations, voltages, and separations times to achieve separation of a specific analytes or group of analytes. Representative separation matrices include, but are not limited to, polymers including polyacrylamides, methacrylates, polysiloxanes, agarose, agar, polyethylene glycol, cellulose, or any other substance capable of forming a meshlike framework or sieve. The separation matrix can be colloids, "polymer solutions," "polymer networks," "entangled polymer solutions," "chemical gels," "physical gels," and/or "liquid gels." More particularly, the separation matrix can be a relatively high-viscosity, crosslinked gel that is chemically anchored to the channel wall ("chemical" gel), and/or a relatively low-viscosity, polymer solution ("physical" gel). The mesh or sieve will work to retain or hinder the movement of large analytes; whereas, small analytes will travel quicker through the mesh. The analytes having similar or identical characteristics such as lengths, molecular weights, or charges, will stack together or travel in bands 414. It will be appreciated that the size of the pores of the separation matrix can be varied to separate different analytes or groups of analytes.

In another embodiment, the inlet 402 or the channel 408 of the microfluidic device can be coated with a substance that specifically binds to a specific analyte or group of analytes. For example, a surface of inlet 402 or the channel 408 can be coated with an antibody that specifically binds directly or indirectly to a specific analyte such as a polypeptide or polynucleotide. Alternatively, a polynucleotide having a predetermined sequence can be attached to a surface of the inlet 402 of the disclosed microfluidic device for binding complementary sequences present in a sample. Suitable polynucleotides are at least about 6 nucleotides, typically about 10 to about 20 nucleotides, even more typically, about 6 to about 15 nucleotides. It will be appreciated that any number of nucleotides can be used, so long as the polynucleotide can specifically hybridize with its complementary sequence or polymers containing its complementary sequence.

Another embodiment provides a microfluidic device having polypeptides attached to an interior surface of a reservoir, tube, or channel. The attached polypeptides can specifically bind to another polypeptide. Exemplary attached polypeptides include, but are not limited to, polyclonal or monoclonal antibodies, fragments of antibodies, polypeptides, for example polypeptides that form dimers with other polypeptides, or polypeptides that specifically associate with other polypeptides or small molecules to form macromolecular complexes or complexes of more than one subunit.

In other embodiments, binding agents are attached to a matrix or resin that is placed inside a reservoir, tube, or channel. The binding matrix or resin can be replaced or recharged as needed. The resins or underlying matrices are generally inert and biologically inactive apart from the binding agent coupled thereto, and can be plastic, metal, polymeric, or other substrate capable of having a binding agent attached thereto. The binding agent can be a polypeptide, small organic molecule, nucleic acid, biotin, streptavidin, carbohydrate, antibody, or ionic compound, fragments thereof, or combinations thereof.

In one embodiment, the inlet 402 receives a plurality of analytes containing a target analyte. Analytes in the sample that are not the target analyte are captured by a binding molecule attached to the surface of the inlet 402, a tube, or channel of the microfluidic device and are immobilized. The target analyte is mobile and is transported through the microfluidic device.

In another embodiment, the target analyte is specifically immobilized by a binding agent such as a polypeptide or polynucleotide. Other analytes are flushed through the microfluidic device. Once the other analytes are separated from the target analyte, the target analyte is released from the binding agent, for example by changing pH, ionic strength, temperature, or a combination thereof. Data from the target analyte can then be captured by the microfluidic device as the target analyte travels through the microfluidic device.

### Reactions

Other embodiments provide reservoirs, wells, or modified tubes or channels, of the microfluidic device that are configured to perform, facilitate, or contain reactions, for example chemical or enzymatic reactions, on a sample containing a plurality of analytes. In one embodiment, a reservoir, channel or tube can be configured to perform polynucleotide amplification or primer extension using, for example, polymerase chain reaction (PCR).

PCR and methods for performing PCR are known in the art. In order to perform PCR, at least a portion of the sequence of the DNA polymer to be replicated or amplified must be known. Short oligonucleotides (containing about two dozen nucleotides) primers that are precisely complementary to the known portion of the DNA polymer at the 3' end are synthesized. The DNA polymer sample is heated to separate its strands and mixed with the primers. If the primers find their complementary sequences in the DNA, they bind to them. Synthesis begins (as always 5' -> 3') using the original strand as the template. The reaction mixture must contain all four deoxynucleotide triphosphates (dATP, dCTP, dGTP, dTTP) a DNA polymerase, for example a DNA polymerase that is not denatured by the high temperature needed to separate the DNA strands. Suitable heat stable DNA polymerases are known in the art and include, but are not limited to Taq polymerase.

Polymerization continues until each newly-synthesized strand has proceeded far enough to contain the site recognized by a flanking primer. The process is repeated with each cycle doubling the number of DNA molecules. Using automated equipment, each cycle of replication can be completed in less than 5 minutes. After 30 cycles, what began as a single molecule of DNA has been amplified into more than a billion copies. It will be appreciated that Reverse Transcriptase PCR is also within the scope of this disclosure.

Other exemplary reactions include fragmenting analytes of a sample. Fragmenting an analyte can be accomplished enzymatically using proteases, peptidases, endonucleases, exonucleases, ribonucleases, physical shearing, sonication, and combinations thereof. Reagents for fragmenting analytes, for example nucleic acids and proteins are known in the art and are commercially available.

### Detection device

Once a sample is aligned and or processed for detection, the sample can be presented to a detector 140. In one embodiment, the detection device 140 is operably coupled to the microfluidic device and can be configured to monitor, collect, transmit, and/or detect data concerning an analyte. A plurality of analytes of the same sequence can be delivered to the detection device 140 in discrete amounts or bands from electrophoretic device 120, for example a microchannel. In still another embodiment, the detection device 140 is electrically insulated from electrophoretic device 120, while optionally remaining in fluid communication with electrophoretic device 120. Electrically insulating the two components allows for different voltage gradients to be applied in the different components. In another embodiment, electrophoretic device 120 is in electrical communication with the detection device 140 such that the voltage gradient maintained in electrophoretic device 120 is also maintained in the detection device 140. In still another embodiment, the polarity of electrophoretic device 120 is maintained in the detection device 140. In another embodiment, the polarity of electrophoretic device 120 is different than the polarity of the detection device 140.

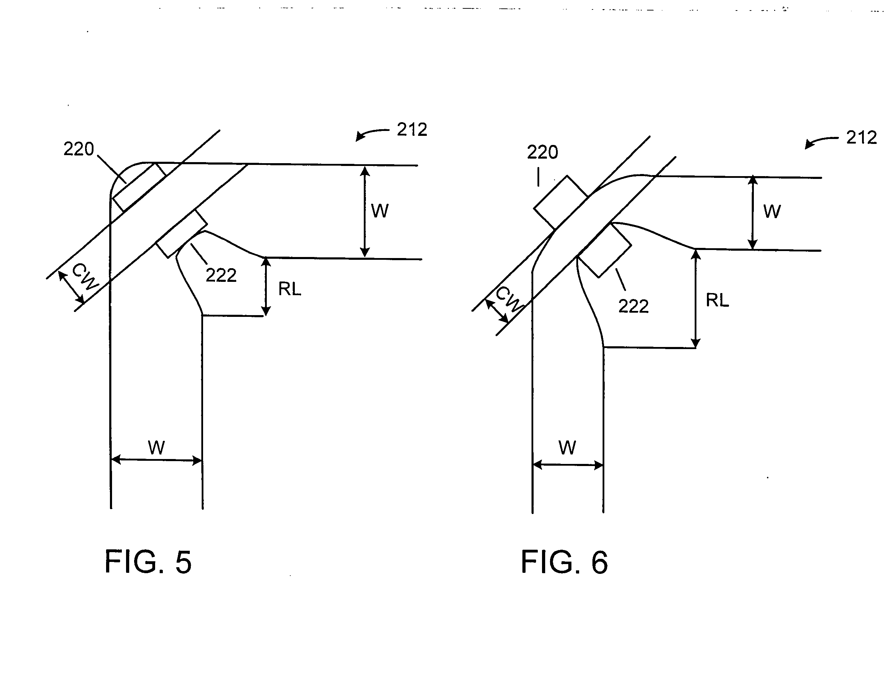

As noted, the detection device 140 also comprises a detector 220, such as an electrode or other sensing device, for collecting data from the analyte as it traverse a microchannel. In one embodiment, the detector can be positioned at a bend or turn in the microchannel (FIGs. 5-6). FIGs 5 and 6 are enlarged views of exemplary serpentine channels 212 of microfluidic device component of system 100. In FIG. 5, the electrodes 220, 222 of a resonant tunnelling electrode 140 are positioned tangent to the interior surface of the arcs forming the turn or bend. The electrodes can be used to narrow the interior diameter of the arcuate portion of the channel 212. FIG. 6 depicts another embodiment in which the detection device is tangent to the interior surface of the channel 212 and does not obstruct the channel 212.

The detectors can be configured to detect or collect different types of data as the analyte passes through a microchannel, including but not limited to, conductivity, ionic current, tunnelling current, temperature, resistance, impedance, fluorescence, radioactivity, or a combination thereof. The data collected, recorded, or transmitted by the detectors can be correlated to specific monomers such that the sequence of monomers forming the polymer can be ascertained. For example, the data obtained from monomers of a specific polymer can be correlated to predetermined values indicative of a specific monomer. The predetermined values can be calculated or determined from polymers of a known sequence of monomers.

### Detectors

As noted above, the microfluidic system 100 comprises at least one detector for collecting data as an analyte or polymer traverse a channel of a disclosed microfluidic device. The data can be used to determine the sequence of monomers forming the polymer. The data can be electromagnetic, conductive, colorometric, fluorometric, radioactive response, or a change in the velocity of electromagnetic, conductive, colorometric, fluorometric, or radioactive component. Detectors can detect a labeled compound, with typical labels including fluorographic, colorometric and radioactive components. Exemplary detectors include resonant tunnelling electrodes, spectrophotometers, photodiodes, microscopes, scintillation counters, cameras, film and the like, as well as combinations thereof. Examples of suitable detectors are widely available from a variety of commercial sources known to persons of skill.

In one embodiment, the detection system is an optical detection system and detects for example, fluorescence-based signals. The detector may include a device that can expose an analyte with an exciting amount of electromagnetic radiation in an amount and duration sufficient to cause a fluorophore to emit electromagnetic radiation. Fluorescence is then detected using an appropriate detector element, e.g., a photomultiplier tube (PMT). Similarly, for screens employing colorometric signals, spectrophotometric detection systems are employed which detect a light source at the sample and provide a measurement of absorbance or transmissivity of the sample.

Other embodiments provide a detection system having non-optical detectors or sensors for detecting particular characteristics or physical parameters of the system or analyte. Such sensors optionally include temperature (useful, e.g., when a reaction produces or absorbs heat, or when the reaction involves cycles of heat as in PCR or LCR), conductivity, potentiometric (pH, ions), amperometric (for compounds that can be oxidized or reduced, e.g., O₂, H₂O₂, I₂, oxidizable/reducible organic compounds, and the like).

Still other detectors are capable of detecting a signal that reflects the interaction of a receptor with its ligand. For example, pH indicators which indicate pH effects of receptor-ligand binding can be incorporated into the device along with the biochemical system (e.g., in the form of encapsulated cells) whereby slight pH changes resulting from binding can be detected. Additionally, the detector can detect the activation of enzymes resulting from receptor ligand binding, e.g., activation ofkinases, or detect conformational changes in such enzymes upon activation, e.g., through incorporation of a fluorophore that is activated or quenched by the conformational change to the enzyme upon activation. Such reporter molecules include, but are not limited to, molecular beacons.

### Resonant Tunnelling Electrode

A representative detector comprises a set of resonant tunnelling electrodes. Resonant tunnelling electrodes are known in the art and are described in US Patent Application Publication No. 2004/0144658 A1 to Flory, which is incorporated herein by reference in its entirety. One embodiment provides a microfluidic device comprising a set of resonant tunnelling electrode. The electrodes 220 and 222 form a representative set of resonant tunnelling electrodes configured to obtain data from analytes such as polymers which travel through a microchannel of the microfluidic device. The term "resonant" or "resonant tunnelling" refers to an effect where the relative energy levels between the current carriers in the electrodes are relatively similar to the energy levels of the proximal analyte or polymer segment. This provides for increased conductivity. Resonant tunnelling electrodes measure or detect tunnelling current, for example from one electrode 220 through an analyte such as a biopolymer to another electrode 222.

The electrodes 220, 222 can be formed in whole or part of one or more of a variety of electrically conductive materials including but not limited to, electrically conductive metals and alloys. Exemplary metals and alloys include, but are not limited to, tin, copper, zinc, iron, magnesium, cobalt, nickel, silver, platinum, gold, and/or vanadium. Other materials well known in the art that provide for electrical conduction may also be employed. When the first electrode 220 is deposited on or comprises a portion of the solid substrate or housing of the microfluidic device, it may be positioned in any location relative to the second electrode 222. The electrodes 220, 222 are typically positioned in an arcuate portion of a microchannel in such a manner that a potential can be established between them. In operation, an analyte such as a biopolymer is generally positioned sufficiently close to the electrodes 220, 222 so specific monomers and their sequence in the biopolymer can be detected and identified. It will be appreciated that the resonant tunnelling electrodes can be fitted to the shape and configuration of a curve or bend in a non-linear portion of a microchannel. Accordingly, the electrodes 220, 222 that can be used with the microchannel 212 can be curved parts of rings or other shapes. The electrodes can also be designed in broken format or spaced from each other. However, the electrodes should be capable of detecting a potential, conductance, or tunnelling current as analytes pass through the space separating the electrodes.

### Methods of Manufacture

The disclosed devices can be fabricated using conventional techniques, including but not limited to spinning a photoresist (positive or negative) onto and silicon substrate. The photoresist is exposed to UV light through a high-resolution mask with the desired device patterns. After washing off the excess unpolymerized photoresist, the silicon wafer is placed in a wet chemical etching bath that anisotropically etches the silicon in locations not protected by photoresist. The result is a silicon wafer in which microchannels are etched. In some embodiments, a glass coverslip is used to fully enclose the channels and holes are drilled in the glass to allow fluidic access. For straighter edges and a deeper etch depth, deep reactive ion etching (DRIE) is an alternative to wet chemical etching. Silicon is a good material for microfluidic channels coupled with microelectronics or other microelectromechanical systems (MEMS). It also has good stiffness, allowing the formation of fairly rigid microstructures, which can be useful for dimensional stability.

### Methods of Use

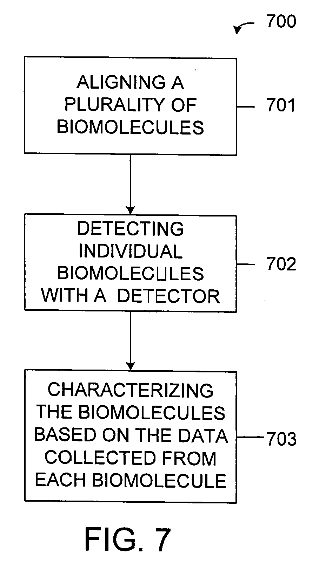

The disclosed devices can be used to characterize an analyte, for example a biomolecule. One embodiment provides an exemplary method for characterizing polynucleotides. As shown in FIG. 7, a plurality of analytes, for example biomolecules, is organized or aligned in step 701 so that each individual analyte or biomolecule is independently presented to a detector. The plurality of analytes can be aligned using, for example, electrophoretic techniques as discussed above. The analytes can be separated or stacked so that analytes can be individually detected by a detector, for example a set of resonant tunnelling electrodes. In step 702, data from each analyte can be obtained. Because multiple analytes can be analyzed, statistically significant data can be collected. In step 703, the data can then be used to characterize the analyte, for example, identify the sequence of a nucleic acid.

Sequencing of polynucleotides has been described (U.S. Pat. No. 5,795,782 to Church et al.; U.S. Pat. No. 6,015,714 to Baldarelli et al., the teachings of which are both incorporated herein by reference in their entireties). In general, nanopore sequencing involves detecting monomers of a polymer as the polymer moves down a voltage gradient established between two regions separated by the detection device 140. The detection device 140 is capable of interacting sequentially with the individual monomer residues of a polynucleotide present in one of the regions. Measurements are continued over time, as individual monomer residues of the polynucleotide interact sequentially, yielding data suitable to infer a monomer-dependent characteristic of the polynucleotide. In some embodiments, the monomer-dependent characterization achieved with the disclosed microfluidic system 100 may include identifying physical characteristics such as, but not limited to, the number and composition of monomers that make up each individual polynucleotide, in sequential order.

The term "sequencing" as used herein refers to determining the sequential order of monomers in a polymer, for example nucleotides in a polynucleotide molecule. Sequencing as used herein comprises in the scope of its definition, determining the nucleotide sequence of a polynucleotide in a de novo manner in which the sequence was previously unknown. Sequencing as used herein also comprises in the scope of its definition, determining the nucleotide sequence of a polynucleotide wherein the sequence was previously known. Sequencing polynucleotides, the sequences of which were previously known, may be used to identify a polynucleotide, to confirm a polynucleotide, or to search for polymorphisms and genetic mutations.

Biopolymers sequenced by the microfluidic system 100 can include polynucleotides comprising a plurality of nucleotide monomers, for example nucleotide triphosphates (NTPs). The nucleotide triphosphates can include naturally occurring and synthetic nucleotide triphosphates. The nucleotide triphosphates can include, but are not limited to, ATP, dATP, CTP, dCTP, GTP, dGTP, UTP, TTP, dTTP, dUTP, 5-methyl-CTP, 5-methyl-dCTP, ITP, dITP, 2-amino-adenosine-TP, 2-amino-deoxyadenosine-TP, 2-thiothymidine triphosphate, pyrrolo-pyrimidine triphosphate, 2-thiocytidine as well as the alphathiotriphosphates for all of the above, and 2'-O-methyl-ribonucleotide triphosphates for all the above bases. Preferably, the nucleotide triphosphates are selected from the group including dATP, dCTP, dGTP, dTTP, dUTP, and combinations thereof. Modified bases can also be used instead of or in addition to nucleotide triphosphates and can include, but are not limited to, 5-Br-UTP, 5-Br-dUTP, 5-F-UTP, 5-F-dUTP, 5-propynyl dCTP, and 5-propynyl-dUTP. Additionally, the nucleotides can be labeled with a detectable label, for example a label that modulates resonant tunnelling current including, but not limited to, metal particles of about 100 nm in diameter or less.

One embodiment provides methods of sequencing a nucleic acid. In the methods, the biochemical components of a sequencing reaction including, but not limted to, a target nucleic acid, a first and optionally, second sequencing primer, a polymerase (optionally including thermostable polymerases for use in PCR, dNTPs, and ddNTPs) are mixed in a microfluidic device under conditions permitting target dependent polymerization of the dNTPs. Polymerization products are separated in the microfluidic device to provide a sequence of the target nucleic acid. Typically, sequencing information acquired by this method is used to select additional sequencing primers and/or templates, and the process is reiterated. Generally, a second sequencing primer is selected based upon the sequence of the target nucleic acid and the second sequencing primer is mixed with the target nucleic acid in a microfluidic device under conditions permitting target dependent elongation of the selected second sequencing primer, thereby providing polymerization products which are separated by size in the microfluidic device to provide further sequence of the target nucleic acid. The nucleic acids are electrophoretically stacked into bands 414 where the polymers in a single band are of uniform charge-to-mass ratio or size and have the same sequence of monomers. Each band_then is analysed by the detection device 140.

It should be emphasized that many variations and modifications may be made to the above-described embodiments. All such modifications and variations are intended to be included herein within the scope of this disclosure and protected by the following claims.

The disclosures in United States patent application no. 11/080,184, from which this application claims priority, and in the abstract accompanying this applications are incorporated herein by reference.

## Claims

1. A microfluidic device (100) including:
a housing;
a serpentine channel (212) disposed in the housing; and
a set of resonant tunnelling electrodes disposed in the serpentine channel (212), wherein the microfluidic device (100) is configured to detect an analyte with the set of resonant tunnelling electrodes.

2. A microfluidic device as claimed in claim 1, wherein the analyte is aligned for detection by the set of resonant tunnelling electrodes.

3. A microfluidic device (100) as claimed in claim 1 or 2, wherein the serpentine channel (212) includes a linear portion in fluid communication with at least one arcuate portion, the linear portion having an inner width W and the arcuate portion having an inner width CW, wherein CW:W is less than 1.0, whereby the width of the arcuate portion is constricted at least in part.

4. A microfluidic device as claimed in claim 3, wherein the set of resonant tunnelling electrodes is tangentially positioned to a curve of the arcuate portion of the channel (212).

5. A microfluidic device as claimed in claim 3 or 4, wherein the set of resonant tunneling electrodes comprises two electrodes separated by a distance approximately equal to CW.

6. A microfluidic device as claimed in any of claims 3 to 5, wherein CW:W is about 0.75 to about 0.25.

7. A method for sequencing an analyte, the method including:
aligning the analyte in a serpentine channel (212) that is disposed in a microfluidic device (100); and
detecting the analyte with a set of resonant tunnelling electrodes.

8. A method as claimed in claim 7, wherein aligning the analytes includes electrophoretically aligning the analyte in the serpentine channel (212).

9. A method as claimed in claim 7 or 8, wherein the serpentine channel (212) includes a linear portion and an arcuate portion.

10. A system including:
a sample preparation device (120); and
a detection device (140) including a serpentine channel (212) in fluid communication with the sample preparation device and a set of resonant tunnelling electrodes disposed in the serpentine channel, wherein the system is configured to detect an analyte with a set of resonant tunnelling electrodes.
